# EUROPEAN PATENT APPLICATION

(11) **EP 4 215 220 A1**
(43) Date of publication of application: **26.07.2023**
(21) Application number: 23020030.5
(22) Date of filing: 20.01.2023
(51) Int. Cl.: A61L 11/00

(54) **LIQUID RECOVERY SYSTEM AND WASTE DISINFECTION APPARATUS**

(30) Priority: 21.01.2022 IT 202200001019
(71) Applicant: Newster System S.r.l., 47853 Cerasolo di Coriano (RN) (IT)
(72) Inventor: Casalboni, Giorgio, 47921 Rimini (RN) (IT); Catapano, Enrico, 47923 Rimini (RN) (IT); Acquaviva, Matteo, 47923 Rimini (RN) (IT)
(74) Representative: Montebelli, Marco

(57) **Abstract**

A liquid recovery system, in particular for a waste disinfection apparatus (2), comprising at least one cooling device (3) for a liquid having at least one inlet opening (6) connectable to the apparatus (2); at least one liquid collection tank (4) having at least one outlet opening connectable to the apparatus (2), and at least one connecting duct (5) between the cooling device (3) and the tank (4).

## Description

This invention relates to a liquid recovery system, in particular for a waste disinfection apparatus, and to a disinfection apparatus.

For the sterilisation or disinfection of waste, in particular infected hospital waste, the prior art recognises the use of an apparatus comprising a steel and cast iron cell, fitted with a lid, into which waste is loaded. In the lower internal part of the cell, a rotor is arranged on which blades are mounted to open the bags of waste and shred the waste. Moreover, situated at the wall of the lower internal part of the cell are fixed blades for shredding and disintegrating the waste.

The rotor is actuated at such a speed as to generate friction and maintain a temperature inside the cell that is sufficient for the sterilisation or disinfection of the waste. The temperature inside the cell is detected and controlled by a sensor which at certain stages of the sterilisation programme triggers the opening of a solenoid valve and the injection of water inside the cell.

The disinfection procedure involves a rotor being actuated until a temperature of about 100°C is reached, with this temperature remaining stable until the water present in the waste has completely evaporated. The temperature then peaks at about 150°C, then mains water is injected to lower the temperature to about 95°C. Once the sterilisation cycle is complete, the material is discharged into a collection system through a discharge outlet.

The water in the treatment cell evaporates due to the high temperature. In the prior art apparatus, the vapours released during the treatment cycle are cooled and then treated in an absolute filter. To cool the vapours, the prior art apparatus uses a liquid separator (demister) consisting of two plastic columns in which the vapor is first condensed by means of the injection of running water and then further cooled. The condensate water is discharged while the cooled vapours enter a filter.

Therefore, the prior art apparatus entails significant water consumption as it requires cooling water during treatment and water to condense the vapour. All of the water is then discharged externally.

The purpose of this invention is to limit water consumption in a waste disinfection apparatus.

According to one aspect of the present invention, a liquid recovery system is provided with the features defined in claim 1.

According to another aspect of the present invention, a cooling unit is also provided with the features defined in claim 3.

According to yet another aspect of the present invention, an apparatus is also provided with the features defined in claim 5.

The present invention will now be described with reference to the accompanying drawings, which show some non-limiting examples of implementations, in which:
- Figure 1 shows a perspective view of a liquid recovery system associated with a waste disinfection apparatus;
- Figure 2 schematically shows a waste disinfection apparatus in a first embodiment;
- Figure 3 schematically shows a cooling unit in a second embodiment.

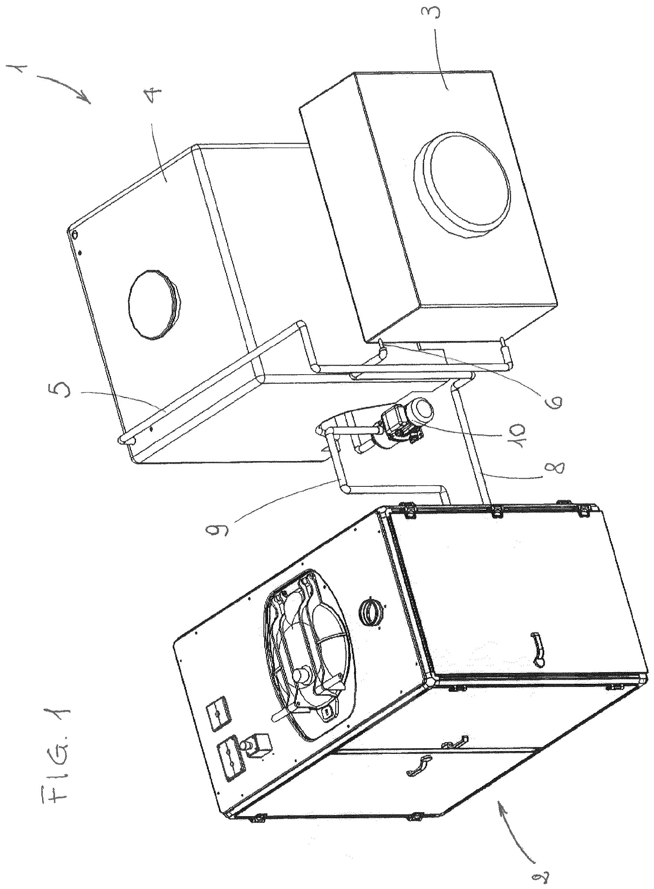

In Figure 1, a liquid - in particular water - recovery system, is, as a whole, indicated with the number 1. System 1 is capable of being associated with a waste disinfection and/or sterilisation unit 2, in particular of hospital waste. The system enables the recovery of vapour cooling water coming from the waste disinfection and/or sterilization unit.

The system 1 comprises at least one liquid cooling device 3, at least one liquid collection tank 4, and at least one connecting duct 5 between the tank 4 and the cooling device 3. The cooling device 3 has at least one liquid inlet opening 6 connectable to a waste disinfection unit 2. The collection tank 4 has at least one liquid outlet opening 7 connectable to the disinfection unit 2.

The recovery system 1 advantageously comprises a first inlet pipe 8 for the liquid entering the cooling device 3 and a second outlet pipe 9 for the liquid exiting the tank 4. The first pipe 8 and the second pipe 9 are capable of being connected to the disinfection unit 2.

The recovery system 1 advantageously comprises at least one pump 10 arranged on the first pipe 8 and/or the second pipe 9.

The cooling device 3 preferably comprises at least one air-water exchanger.

The recovery system 1 makes it possible to limit water consumption by reusing the water that cools the vapour deriving from the treatment of waste in the disinfection apparatus.

The water used to cool the vapour is conveyed into the cooling device 3 through the pipe 8, for example, by means of a pump. In the cooling device 3, the water temperature is lowered and the water is then transferred into the tank 4 through the duct 5. The tank 4 is interposed between the cooling device 3 and the cooling unit 11. Cold water is then conveyed into the pipe 9 for reuse in a vapour cooling unit 11.

The collection tank 4 allows a certain amount of liquid to be available at a constant temperature so as to reduce the power of the cooling device 3. The cooling device 3 may comprise for instance, a fan.

Advantageously, the recovery system circuit is not pressurised.

Advantageously, the cooling unit 11 comprises at least one heat exchanger 12 of the vapour-water type. The heat exchanger 12 preferably comprises at least one vessel, particularly made of steel, with a tube bundle arranged inside of it. The heat exchanger has an inlet 13 for the vapours to be cooled, which is connectable to the disinfection unit 2. The vapours from the disinfection unit 2 circulate in the tube bundle, while the cooling water circulates outside the tube bundle. Condensed vapour is discharged outside the heat exchanger 12. The heat exchanger 12 has an inlet 14 for cooling water passing outside the tube bundle and an outlet 15 for water that has passed through and been heated by the heat exchanger. The collection tank 4 is interposed between the cooling device 3 and the cooling water inlet 14 of the heat exchanger 12. The cooling water inlet 14 of the heat exchanger 12 is connected to the collection tank 4 by means of the pipe 9. The inlet 14 for the cooling water coming from the collection tank 4 is connected to the pipe 9 and the outlet 15 for the heated water is connected to the cooling device 3 by means of the pipe 8. In this way, cooling water is recycled, which limits its consumption.

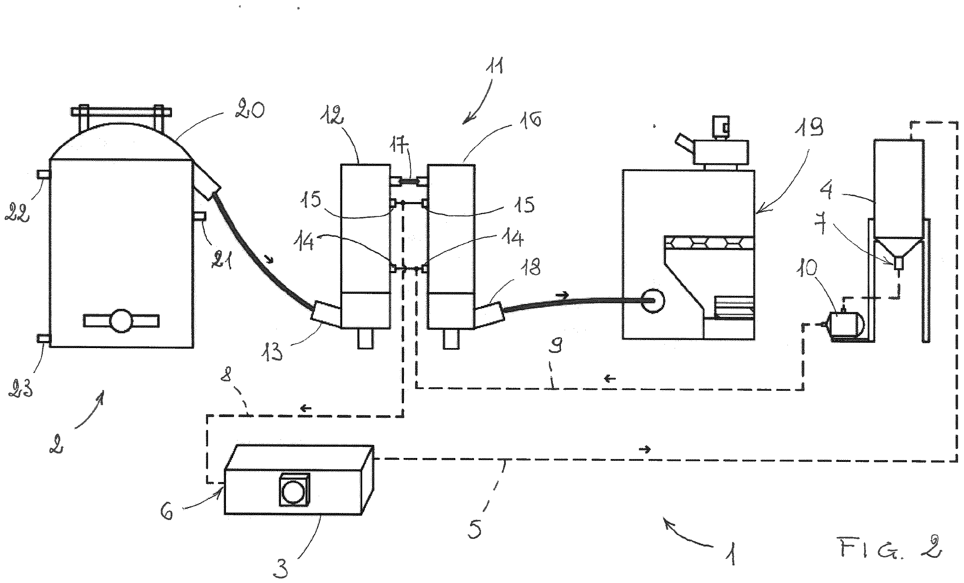

In the embodiment shown in Figure 2, the cooling unit 11 comprises two heat exchangers 12, 16, in particularly of cylindrical shape, connected by a pipe 17 in such a way that the vapour from the disinfection unit 2 passes into the first heat exchanger 12 wherein the initial cooling is carried out and then into the second heat exchanger 16. The second heat exchanger 16 has an outlet 18 from which vapour passes into a unit 19 provided with at least one filter. The water used for cooling, coming from the two heat exchangers 12, 16, is conveyed to the cooling device 3. Water passes from the cooling device 3 into the collection tank 4, from whence it is drawn by the pump 10 and conveyed to the heat exchangers 12, 16 to be reused. Figure 2 schematically shows a disinfection and/or sterilisation apparatus in a preferred embodiment.

The disinfection apparatus comprises a disinfection unit 2 that features a treatment cell 20 having at least one first liquid inlet opening 21, in particular for water, at least one second inlet opening 22 for air, and at least one temperature sensor 23. In the disinfection and/or sterilisation apparatus, the waste treatment process involves water being added inside the cell 20 after a peak of about 150°C has been reached.

The water introduced into the cell 20 evaporates instantly. The moisture contained in the material also evaporates due to the high temperature. The vapours are aspirated and cooled in a heat exchanger.

The water recovery system 1 is connected to the treatment cell 20 by means of the cooling unit 11. The cooling unit 11 comprises at least a first heat exchanger 12 of the vapour-water type. The heat exchanger 12 preferably comprises at least one vessel, in particular made of steel, with a tube bundle arranged inside of it.

In the embodiment shown in Figure 2, the apparatus comprises two heat exchangers 12, 16. The first heat exchanger 12 has an inlet 13 connected to the treatment cell 20 through which vapours are conveyed. The second heat exchanger 16 has an outlet 18 connected to a filtering device 19 to discharge the cooled vapours. The two heat exchangers 12, 16 are connected to each other by a pipe 17 for vapours to pass. The collection tank 4 is interposed between the cooling device 3 and the cooling water inlet 14 of the heat exchangers 12, 16. The first inlet pipe 8 of the cooling device 3 is connected to the cooling liquid outlet 15 of the two heat exchangers 12, 16 and the second outlet pipe 9 for liquid from the tank 3 is connected to the cooling liquid inlet 14 of the two heat exchangers 12, 16.

The recovery system allows the water used for vapour cooling to be recycled, reducing its consumption by up to 90%.

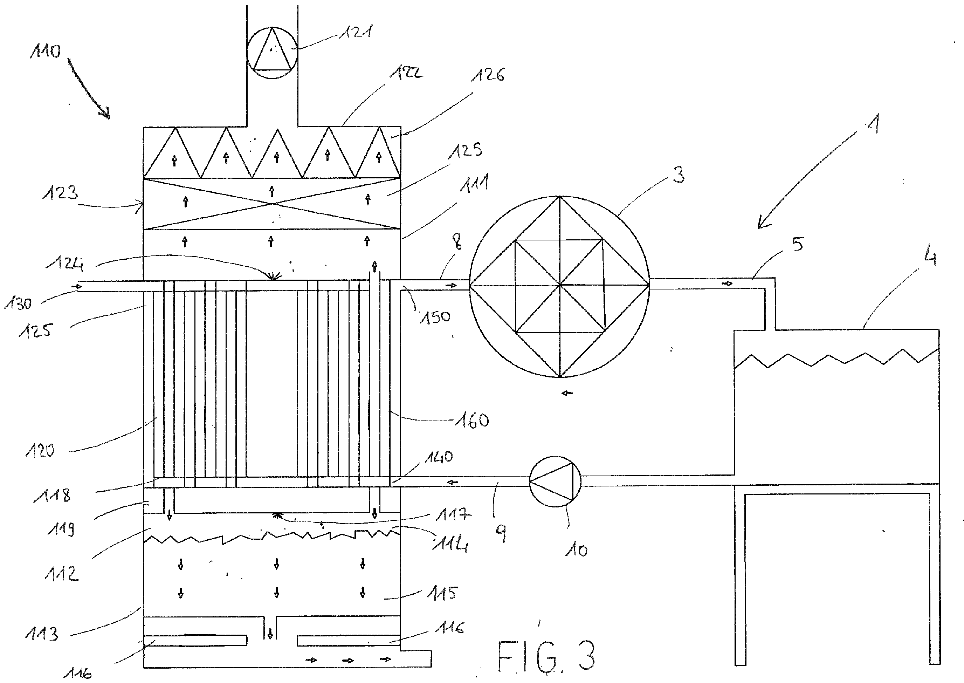

According to another aspect of the present invention, with reference to Figure 3, a cooling unit 110 comprises a vessel 111 and at least one heat exchanger 120 of the vapour-water type arranged within the vessel 111.

The cooling unit 110 comprises a filtering unit 112 for the condensate coming from the heat exchanger 120. The filtering unit 112 is preferably arranged in the lower area 113 of the vessel 111. Advantageously, the filtering unit 112 comprises a carbon filter 114, in particular a granular carbon filter.

A condensate collection tank 115 is preferably arranged downstream from the filtering unit 112. At least one UV lamp 116, in particular a UV-C lamp, is advantageously arranged downstream from the collection tank 115.

In one preferred embodiment, a device for injecting 117 a product for the reduction of dissolved organic compounds, particularly an enzymatic product, is arranged inside the vessel 111. The injection device 117 is preferably arranged between the lower portion 118 of the heat exchanger 120 and the filtering unit 112.

The vessel 111 preferably has an air inlet 119 arranged in the lower area 113 of the vessel 111, downstream from the heat exchanger 120, to ensure the correct flow rate in the filtration system and to increase the capacity of the vapour condensation. The cooling unit 110 advantageously comprises a vapour suction unit 121, such as a fan. The suction unit 121 is preferably arranged in the upper area 122 of the vessel 111.

The cooling unit 110 advantageously comprises a filtering unit 123 for the cooled vapours. The filtering unit 123 is preferably arranged in the lower area 122 of the vessel 111.

In one preferred embodiment, a device for spraying 124 a product for the reduction of volatile residual organic compounds, particularly an enzymatic product, is arranged inside the vessel 111, in particular in the upper area 122. The spraying device 124 is preferably between the upper portion 125 of the exchanger 120 and the filtering device 123.

In a preferred embodiment, the filtering unit 123 comprises at least one HEPA filter 125, for example HP14, and/or one carbon filter 126, for example of carbon block type.

Advantageously, the exchanger 120 is a countercurrent shell and tube exchanger. The cooling unit 110 is connectable to the treatment cell. The cooling unit 110 is connectable to the liquid recovery system 1.

In particular, the heat exchanger 120 has a vapour inlet 130. The vapour may come from the treatment cell of a disinfection unit 2.

The first inlet pipe 8 of the cooling device 3 is connected to the cooling liquid outlet 150 of the heat exchanger 120 and the second outlet pipe 9 for liquid from the tank 3 is connected to the cooling liquid inlet 140 of the heat exchanger 120.

In the embodiment shown in Figure 3, the cooling unit comprises two heat exchangers 120, 160 arranged inside the vessel 111. The first heat exchanger 120 and the second heat exchanger 160 have at least one vapour inlet 130. The inlet 130 may be connected to the treatment cell of the disinfection unit 2.

The first heat exchanger 120 and the second heat exchanger 160 have at least one cooling liquid outlet 150. The first inlet pipe 8 of the cooling device 3 is connected to the cooling liquid outlet 150 of the heat exchangers 120, 160. The second inlet pipe 9 of the tank 3 is connected to the cooling liquid inlet 140 of the heat exchangers 120, 160.

In the cooling unit 110 shown in Figure 3, the vapour produced during the sterilisation cycle by heating waste in the treatment cell, particularly in the heating step from room temperature to temperature T=100°C and in the cooling step from temperature T=150°C to temperature T=90°C, is continuously removed by the suction device 121, such as a fan.

Vapour enters the vapour/water cooling unit 110 comprising two shell and tube heat exchangers 120, 160, where a coolant liquid is recirculated at a constant temperature and countercurrent by the means of the pump 10 and the collection tank 4. The constant temperature is maintained thanks to the dissipation of heat by the cooling device 3, such as an radiator with the appropriate power.

The vapour condenses in the tube bundle and never comes into contact with the coolant, which is continuously recirculated. The condensate is discharged into a granular activated carbon filter 114, where an enzymatic product for the reduction of dissolved organic compounds is also injected. The condensate percolates through the carbon and passes, filtered, into the tank 115 below, where it is irradiated with UV-C 116 lamps before being discharged to the sewer.

The aeriform emission that remains, due to the incomplete condensation of the vapour produced, is sprayed with the enzyme product for reducing the residual volatile organic compounds, and is then filtered by means of a HEPA filter 125, for example HP14, and a carbon filter 126, for example of carbon block type, before being released into the atmosphere. Therefore, the cooling unit ensures that the limits for aeriform and liquid emissions are altogether complied with.

## Claims

1. A liquid recovery system, in particular for a waste disinfection and/or sterilisation apparatus (2), the system comprising at least one cooling device (3) for a liquid having at least one inlet opening (6) connectable to a disinfection unit (2); at least one liquid collection tank (4) having at least one outlet opening connectable to the disinfection unit (2), and at least one connecting duct (5) between the cooling device (3) and the tank (4).

2. The system according to claim 1, **characterised in that** it comprises a first inlet pipe (8) for the liquid entering the cooling device (3) and a second outlet pipe (9) for the liquid exiting the tank (4).

3. A cooling unit, in particular for a waste disinfection and/or sterilisation apparatus (2), comprising at least one first heat exchanger (12; 120) connected to a liquid recovery system according to claim 1 or 2, **characterised in that** the heat exchanger (12; 120) has at least one vapour inlet opening (13; 130) connectable to the disinfection unit (2); at least one liquid inlet (14; 140) connected to the collection tank (4) and at least one liquid outlet (15; 150) connected to the cooling device (3).

4. The cooling unit according to claim 3, **characterised in that** it comprises a second heat exchanger (16; 160) having at least one vapour inlet opening connectable to the first heat exchanger (12; 120); at least one liquid inlet (14; 140) connected to the collection tank (4) and at least one liquid outlet (15; 150) connected to the cooling device (3).

5. The cooling unit according to any one of claims 3 or 4, **characterised in that** it comprises at least one device (117; 124) for injecting and/or spraying a product for the reduction of organic compounds, particularly an enzymatic product.

6. The cooling unit according to any one of the preceding claims 3 to 5, **characterised in that** it comprises at least one vapour suction device (121).

7. The cooling unit according to any one of the preceding claims 3 to 6, **characterised in that** it comprises at least one vapour filtering unit (123).

8. The cooling unit according to any one of the preceding claims 3 to 7, **characterised in that** it comprises at least one vapour filtering unit (112) for the condensation coming from the exchanger (120).

9. The cooling unit according to any one of the preceding claims 3 to 7, **characterised in that** it comprises at least one UV lamp (116), in particular UV-C, to irradiate the condensation coming from the exchanger (120).

10. A waste disinfection and/or sterilisation apparatus, in particular for hospital waste, comprising a treatment cell (20) having at least one first liquid inlet opening (21), in particular for water, at least one second inlet opening (22) for air, and at least one temperature sensor (23), wherein the apparatus comprises at least one cooling unit according to any one of the preceding claims 3 to 9.

11. A waste disinfection and/or sterilisation apparatus, in particular for hospital waste, comprising a treatment cell (20) having at least one first liquid inlet opening (21), in particular for water, at least one second inlet opening (22) for air, and at least one temperature sensor (23), wherein the apparatus comprises at least one heat exchanger (12) for cooling the vapour arriving from the treatment cell (20), and a liquid recovery system (1) according to claim 1 or 2 connected to the heat exchanger (12).

12. The apparatus according to claim 11, **characterised in that** the heat exchanger (12) has at least one opening (13) connected to the treatment cell (20) for entry of the vapour to be cooled; at least one liquid inlet (14) connected to the collection tank (4) and at least one liquid outlet (15) connected to the cooling device (3).

13. The apparatus according to claim 12, **characterised in that** it comprises a second heat exchanger (16) having at least one opening connected to the first heat exchanger (12) for entry of the vapour to be cooled; at least one liquid inlet (14) connected to the collection tank (4) and at least one liquid outlet (15) connected to the cooling device (3).
